(19) Europäisches Patentamt
European Patent Office
Office européen des brevets

(11) EP 1 438 079 B1

(12) EUROPÄISCHE PATENTSCHRIFT

(45) Veröffentlichungstag und Bekanntmachung des Hinweises auf die Patenterteilung:
**20.04.2005 Patentblatt 2005/16**

(21) Anmeldenummer: **02787500.4**

(22) Anmeldetag: **24.10.2002**

(51) Int Cl.[7]: **A61L 24/00**, A61L 24/08, A61L 24/04, C08L 5/08, C08L 5/02

(86) Internationale Anmeldenummer:
**PCT/EP2002/011880**

(87) Internationale Veröffentlichungsnummer:
**WO 2003/035122 (01.05.2003 Gazette 2003/18)**

(54) **ZUSAMMENSETZUNG AUS EINEM AMINOGRUPPEN TRAGENDEN POLYMER UND EINEM ALDEHYD MIT MINDESTENS DREI ALDEHYDGRUPPEN**

COMPOSITION CONSISTING OF A POLYMER CONTAINING AMINO GROUPS AND AN ALDEHYDE CONTAINING AT LEAST THREE ALDEHYDE GROUPS

COMPOSITION CONTENANT UN POLYMERE PORTANT DES GROUPES AMINO ET UN ALDEHYDE COMPORTANT AU MOINS TROIS GROUPES ALDEHYDE

(84) Benannte Vertragsstaaten:
**DE ES FR GB IT**

(30) Priorität: **24.10.2001 DE 10152407**

(43) Veröffentlichungstag der Anmeldung:
**21.07.2004 Patentblatt 2004/30**

(73) Patentinhaber: **Aesculap AG & Co. KG**
**78532 Tuttlingen (DE)**

(72) Erfinder:
- **GOLDMANN, Helmut**
  **78532 Tuttlingen (DE)**
- **WEGMANN, Jürgen**
  **78333 Stockach (DE)**

(74) Vertreter: **Patentanwälte**
**Ruff, Wilhelm, Beier, Dauster & Partner**
**Kronenstrasse 30**
**70174 Stuttgart (DE)**

(56) Entgegenhaltungen:
**EP-A- 0 413 136**
**WO-A-99/01143**
**US-A- 6 051 648**
**US-A- 6 165 488**

- **HARRIS J M ET AL: "SYNTHESIS AND CHARACTERIZATION OF POLY(ETHYLENE GLYCOL) DERIVATIVES" JOURNAL OF POLYMER SCIENCE, INTERSCIENCE PUBLISHERS, XX, Bd. 22, 1984, Seiten 341-352, XP001020419**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 438 079 B1

Printed by Jouve, 75001 PARIS (FR)

## Beschreibung

Die Erfindung betrifft eine Zusammensetzung aus mindestens zwei, insbesondere zwei, biokompatiblen, untereinander chemisch vernetzbaren Komponenten, insbesondere zum Verkleben von biologischem Gewebe, umfassend mindestens folgende Komponenten:

a) wässrige Lösung mindestens eines aminogruppentragenden Polymers
b) wässrige Lösung mindestens eines Aldehyds mit mindestens drei Aldehydgruppen.

In der Chirurgie werden zum Zusammenfügen von getrennten Gewebeteilen hauptsächlich Nahtmaterialien und Klammern verwendet. Diese Techniken stoßen jedoch vor allem in der minimal invasiven Chirurgie, zu welcher unter anderem die Laparoskopie, die Thorakokospie, die Arthroskopie, die Kardiochirurgie sowie die intraluminale Endoskopie zählen, auf ihre Grenzen. In diesen Bereichen ist die Anwendung von Gewebeadhäsiven und Sealants einfacher, schneller und sicherer. Mehrere Patente beschreiben synthetische und natürliche Polymer- oder Makromersysteme, die zum Verkleben von Weichgewebe, zum Abdichten von Luft- und Flüssigkeitsleckagen in Organen und Blutgefäßen angewendet werden können.

Die auf dem Markt kommerziell erhältlichen Fibrinkleber bestehen unter anderem aus humanen oder/und bovinen Plasmaproteinen, die hinsichtlich der Übertragung von Infektionen ein erhebliches Gesundheitsrisiko darstellen. Zudem ist ihre Haftkraft oft unzureichend. Im Vergleich zu Fibrinklebern weisen Hydrogele deutlich höhere cohäsive wie auch adhäsive Eigenschaften auf. Besonders geeignet sind Zusammensetzungen, die in flüssigem Zustand auf das Gewebe aufgebracht werden können und dann durch Ausbildung kovalenter Bindungen innerhalb kurzer Zeit aushärten. Die in situ Aushärtung beruht in der Regel auf der Vernetzung von Makromersystemen und kann durch radikalische Polymerisation oder durch chemische Reaktion mit bi- oder multifunktionellen Vernetzungsreagenzien erfolgen.

Die radikalische Vernetzung kann durch Licht- oder Wärmequellen sowie über oxidative Radikalbildung mit anorganischen Persulfaten oder Enzymen ausgelöst werden. In den US-Patenten 6,156,345, Chudzik et al., US 6,083,524, Sawhney et al. und US 6,060,582, Hubbel et al., werden synthetische Makromere mit radikalisch polymerisierbaren Endgruppen beschrieben, deren Polymerisation durch Bestrahlung mit UV-Licht in situ auf dem Gewebe initiiert wird. Neben synthetischen Polymeren können auf diese Weise auch viskose Lösungen von Kollagen und Kollagenderivaten vernetzt werden (US 6,183,498 B1, Devore et al., US 5,874,537 Kelman et al.). Aufgrund der zusätzlich benötigten Lichtquelle ist die Technik sehr aufwendig und teuer. Als Alternative zur UV-Aktivierung kann eine Polymerisation auch mit Hilfe einer Wärmequelle ausgelöst werden. Die erforderlichen Temperaturen beschädigen allerdings gesunde Zellen im Gewebe und töten diese häufig ab. Prinzipiell ist die Beschädigung von gesundem Gewebe bei den meisten radikalischen Polymerisationen ein Problem, da diese exotherm verlaufen, d.h., während der Polymerisation wird Wärme an die Umgebung abgegeben.

Als Alternative zur radikalischen Polymerisation können Makromere auch über reaktive Gruppen chemisch vernetzt werden. Vor allem Carbonyl- wie auch bestimmte Carboxylreaktionen besitzen bezüglich der Kinetik die gewünschten Eigenschaften, um eine schnelle Gelierung der Komponenten zu gewährleisten. In US-Patent 6,051,648, Rhee et al., werden synthetische Polymere mit N-Hydroxysuccinimid aktivierten Carboxylgruppen beschrieben, die unter Abspaltung des N-Hydroxysuccinimids mit nukleophilen multifunktionellen Polymeren vernetzen. Durch die fehlende Stabilität der aktivierten Carboxylgruppen in wässriger Lösung müssen hierbei vorgeformte Patchs angewendet werden, was gerade in der minimal invasiven Chirurgie erhebliche Nachteile mit sich bringt.

Freie Lysineinheiten in Polypeptiden und Proteinen bilden durch die Reaktion mit Di- oder Polyaldehyden Schiff'sche Basen aus. Kowanko beschreibt in US-Patent 5,385,606 eine adhäsive Zusammensetzung bestehend aus humanen oder tierischen Proteinen und einem Di- oder Polyaldehyd, wobei die Vernetzung bevorzugt mit Glutaraldehyd durchgeführt wird. Die Verwendung von Glutaraldehyd ist jedoch kritisch. Vries et al. (Abstract Book of the Second Annual Meeting of the WHS, Richmond, USA p. 51, 1992) konnten nachweisen, daß mit Glutaraldehyd vemetzte Gelatine toxische Wirkung auf Zellen hatte, was bei reiner Gelatine nicht der Fall ist.

In US-Patent 6,165,488 hingegen beschreiben Tardy et al. einen biokompatiblen Gewebekleber bestehend aus einer wässrigen Kollagenlösung und einer wässrigen Polyaldehydlösung und vermeidet somit die Verwendung von kleinen toxischen Molekülen zur Vernetzung. Ein Gewebekleber aus oxidiertem Dextran oder Stärke und modifizierter Gelatine wird auch von Mo et al. in J. Biomater, Sci. Polymer Edn. 2000, 11, 341-351 beschrieben. Dextran ist in vielen Medizinprodukten enthalten und wird zum Beispiel in Wunddressings in oxidierter Form als vernetzende Komponente verwendet (Schacht et al, US-Patent 6,132,759). Die makromolekularen Vernetzungsreagenzien werden dabei durch Oxidation von Dextran oder Stärke mit Natriumperiodat hergestellt. Diese Reaktion wurde unter anderem von Bernstein et al. (Natl. Cancer Inst. 1978, 60, 379-384) beschrieben und ist Stand der Technik. Die Verwendung von Kollagen in der Medizin ist dagegen bezüglich der Infektionsgefahr kritisch, besonders im Hinblick auf BSE und Creutzfeld-Jakob Erkrankungen. Zudem können durch Eiweißstoffe Immunreaktionen im Körper ausgelöst werden.

Chitin ist in der Natur ein weitverbreitetes lineares, stickstoffhaltiges Polysaccharid und bildet den Hauptbestandteil des Außenskelettes von Gliederfüßern (Maikäferflügel, Hummer- und Garnelenschalen). In konzentrierter Natronlauge entsteht aus Chitin das Deacetylierungsprodukt Chitosan, welches im Gegensatz zum Chitin freie Aminogruppen besitzt und in schwach saurem, wässrigem Medium löslich ist. Das Degradationsverhalten von reinem und mit Glutaraldehyd behandelten Chitosan sowie die akute Toxität und die hämostatische Wirkung von Chitosan wird von Rao et al. beschrieben (J. Biomed. Mater. Res. 1997, 34, 21-28). Aufgrund der antimikrobiellen und hämostatischen Wirkung in Kombination mit ihrer hohen Biokompatibilität sind Chitosan und Chitin vielversprechende Substanzen für medizinische Produkte. In US-Patent 6,124,273 werden Proteine in Chitosanschwämme eingearbeitet und die Zusammensetzung bei äußerlichen Wunden eingesetzt. Die Chitosanschwämme setzen dabei die Proteine frei und beschleunigen die Wundheilung. Ono et al. beschreiben einen biologischen Gewebekleber aus photovernetztem Chitosan (K. Ono, et al., J. Biomed. Mater. Res. 2000, 49, 289-295). Die Vernetzung erfolgt durch Bestrahlung mit UV-Licht. Diese teure und aufwendige Technik hat sich in der Praxis, wie bereits erwähnt, nicht durchgesetzt. Zudem ist die Haftkraft dieses Adhäsives unzureichend, sie liegt im Bereich der Fibrinkleber.

Der Erfindung liegt die Aufgabe zugrunde, eine Zusammensetzung zu schaffen, welche die genannten Nachteile des Standes der Technik überwindet, insbesondere die Gefahr der Übertragung von Infektionskrankheiten vermeidet.

Diese Aufgabe wird erfindungsgemäß durch eine Zusammensetzung mit den Merkmalen des Anspruchs 1 gelöst. Bevorzugte Aus- bzw. Weiterbildungen der erfindungsgemäßen Zusammensetzung sind in den Unteransprüchen gekennzeichnet.

Durch die Tatsache, daß die erfindungsgemäße Zusammensetzung frei von Eiweiß ist, ist die Gefahr einer Übertragung von Infektionskrankheiten, die bei einer Verwendung von Eiweiß (z.B. Kollagen) gegeben ist, ausgeschaltet. Dies ist insbesondere im Hinblick auf eine mögliche Übertragung von BSE-Erregern auf den Menschen ein großer Vorteil gegenüber den, im Stand der Technik beschriebenen eiweißhaltigen Zusammensetzungen. Zudem ist auch die Gefahr von eiweißbedingten Immunreaktionen bei der erfindungsgemäßen, eiweißfreien Zusammensetzung gebannt.

Ein weiterer Vorteil der erfindungsgemäßen Zusammensetzung besteht darin, daß die Gelierung der Komponenten spontan erfolgt und keine zusätzlichen Energiequellen erforderlich sind. Dadurch ist die Applikation vereinfacht und verläuft gewebeschonend, da das gesunde Gewebe nicht z. B. durch übermäßig hohe Wärmeenergie beeinträchtigt wird.

Ein weiterer Vorteil der Erfindung ist es, daß die Komponenten in wässrigem Medium appliziert werden können und dadurch eine bessere Bedeckung der Wundfläche gewährleistet ist als dies beispielsweise bei vorgeformten Patches der Fall ist (vgl. z.B. US 6,051,648).

Bei einer besonders bevorzugten Ausführungsform der erfindungsgemäßen Zusammensetzung sind der Aldehyd und das aminogruppentragende Polymer bei Körpertemperatur miteinander vernetzbar. Dadurch können die oben genannten zusätzlichen Energiequellen vermieden werden, was wiederum Gewebeschädigungen vermeidet.

Vorzugsweise ist das aminogruppentragende Polymer von einem biologisch abbaubaren Naturstoff abgeleitet. Besonders bevorzugt ist es, daß das aminogruppentragende Polymer ein Polysaccharid, insbesondere ein modifiziertes Polysaccharid, bei dem die Aminogruppen durch Deacetylierung freigesetzt sind, ist. Bei einer besonders bevorzugten Ausführungsform der erfindungsgemäßen Zusammensetzung ist das aminogruppentragende Polymer ein mindestens teilweise deacetyliertes Chitin mit einem Deacetylierungsgrad von 50 bis 100 %, vorzugsweise 60 bis 90 %, insbesondere 70 bis 80 %. Durch die Deacetylierung werden die Acetamidgruppen im Chitin in Aminogruppen umgewandelt. Dies bewirkt u.a. wiederum, daß der Abbau im Körper langsamer verläuft als bei (nicht deacetyliertem) Chitin. Besonders bevorzugt ist es, wenn das aminogruppentragende Polymer Chitosan ist. Chitosan hat eine blutgerinnende Wirkung. Deacetyliertes Chitin, insbesondere Chitosan, wird vorzugsweise in wasserlöslicher Salzform eingesetzt (Chlorid, Acetat, Glutamat).

Bei einer weiteren Ausführungsform der erfindungsgemäßen Zusammensetzung ist das Aminogruppen tragende Polymer ein synthetisches Polymer, insbesondere ein nierengängiges Polymer. Dies hat den Vorteil, daß eine einfache Ausscheidung des aminogruppentragenden Polymers über den Urin möglich ist. Vorteilhafterweise ist das synthetische Polymer ein modifizierter aminogruppentragender Polyvinylalkohol, vorzugsweise mit einem Molekulargewicht $\leq$ 50.000, insbesondere < 50.000, vorzugsweise $\leq$ 20.000, insbesondere < 20.000.

Beispiele für die Modifizierung von Polyvinylalkohol sind die Veresterung von Polyvinylalkohol mit Aminosäuren, die Veresterung mit Dicarbonsäuren bzw. -anhydriden verbunden mit einer Amidbildung mit mehrwertigen Aminen, insbesondere Diaminen, und die Bildung zyklischer Acetale.

Der Modifizierungsgrad kann beliebig eingestellt werden und ist nicht auf die Kettenenden beschränkt wie zum Beispiel bei PEG oder Polyhydroxyalkanoaten. Als weitere multifunktionelle Polymere mit freien Hydroxygruppen stehen auch Polysaccharide wie Dextran, Cellulose, Chitosan, Hyaluronsäure, Alginsäure, Stärke, Agar, Chitin und Chondroitinsulfat zur Verfügung.

Beispiele für Modifikationen von Polyvinylalkohol (PVA)

a) Retrosynthese von Alanin modifiziertem PVA

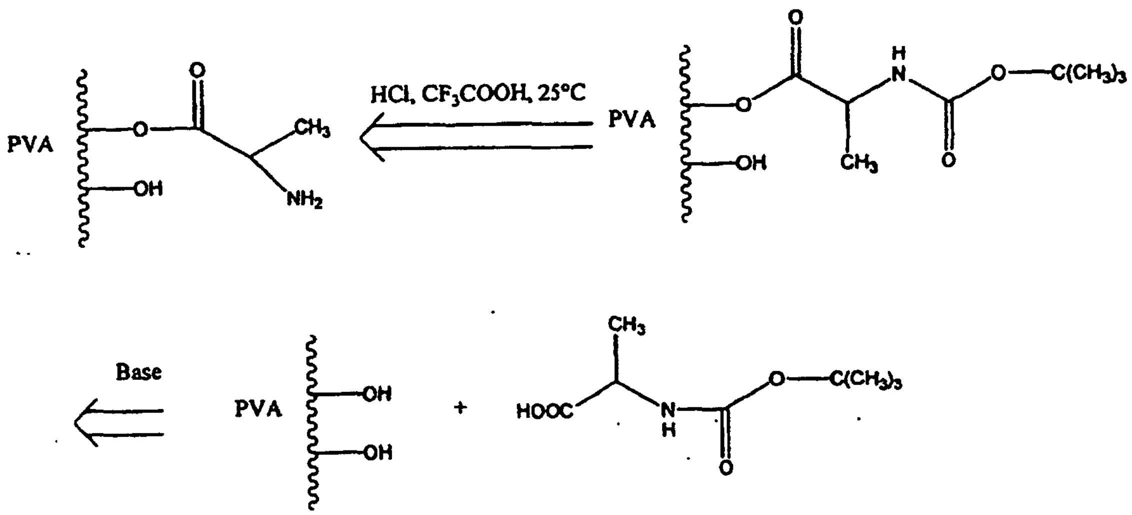


Die Anbindung von Aminosäuren an Polyvinylalkohol erfolgt in zwei Schritten. Zuerst wird der Alkohol mit einem BOC geschützten Alanin verestert. Als Katalysator wird eine Base hinzugefügt. Die Reaktion muss in wasserfreiem Lösungsmittel durchgeführt werden. Nach erfolgreicher Anbindung kann die BOC Schutzgruppe unter milden Bedingungen bei Raumtemperatur mit Trifluoressigsäure abgespalten werden. Prinzipiell kann jede beliebige Aminosäure auf diese Weise angebunden werden, bevorzugt sind jedoch Aminosäuren mit zusätzlichen Thiol- oder Hydroxygruppen, wie z.B. Cystein, Serin, Threonin, Tyrosin, besonders bevorzugt sind Aminosäuren mit weiteren Aminogruppen wie z.B. Asparagin, Lysin, Glutamin, Arginin oder Tryptophan. Ebenso ist die Anbindung einer Mischung aus den erwähnten Aminosäuren denkbar.

Vorteil:

- keine Amidbindungen, Esterbindungen sollten hydrolytisch spaltbar sein
- Abbauprodukt ist eine Aminosäure (toxikologisch unbedenklich).

b) Retrosynthese mit Bernsteinsäureanhydrid und Diamin

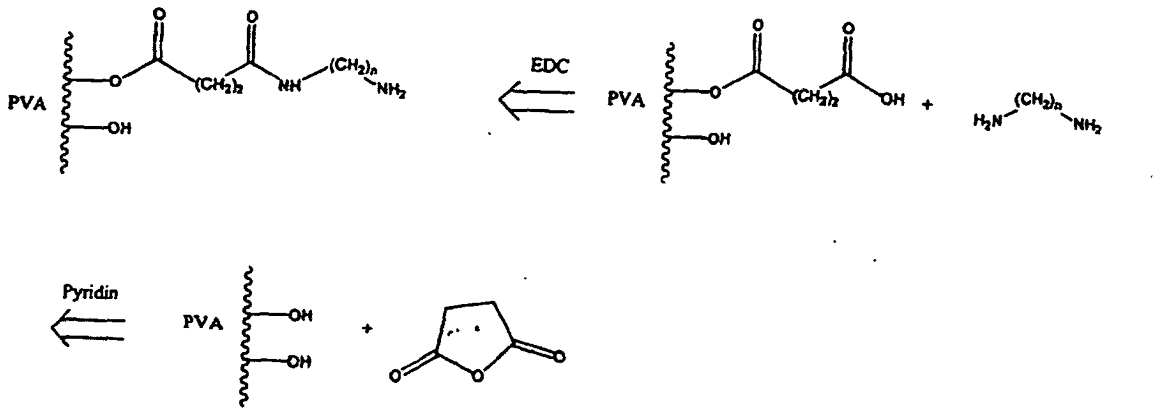


Die Anbindung von freien Aminogruppen an Polyvinylalkohol über zyklische Disäureanhydride erfolgt ebenfalls in zwei Stufen. Im ersten Schritt wird das Anhydrid mit Hilfe einer katalytisch wirkenden Base EDC an den Alkohol gebunden. Anschließend erfolgt die Umsetzung mit einem Diamin. Das Diamin sollte im Überschuss eingesetzt werden, um eine Vernetzung des Polyvinylalkohols während der Reaktion zu vermeiden. Als Disäureanhydride können u.a. auch Maleinsäureanhydrid, Adipinsäureanhydrid oder Glutarsäureanhydrid verwendet werden.

Vorteil:

- Ausgangssubstanzen sind sehr günstig
- Anbindung an PVA durch Esterbindung kann leicht abgebaut werden
- Diamine könnten toxikologisch problematisch sein (möglicher Ersatz durch Triethylenglycoldiamin) ($NH_2$-$C_2H_4$-O-$C_2H_4$-O-$C_2H_4$-$NH_2$))

c) Einführung von terminalen Aminogruppen über zyklische Acetale

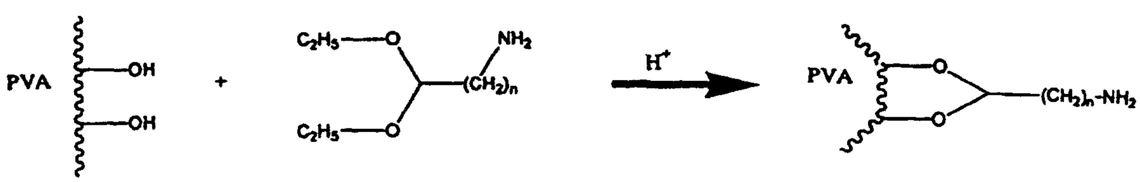


Über Acetalbindungen lassen sich in einem Schritt terminale Aminogruppen in den Polyvinylalkohol einbringen. Die Bildung des zyklischen Acetals ist dabei energetisch bevorzugt. Die Kettenlänge des Spacers kann variiert werden, bevorzugt ist n ≤ 4, besonders bevorzugt ist n = 1.

Vorteile:

- Einführung der Aminogruppe in einem einzigen Syntheseschritt
- Keine Schutzgruppenchemie, eine Vernetzung während der Reaktion ist nicht zu erwarten.

Es können auch Kombinationen von aminogruppentragenden Polysacchariden und aminogruppentragenden Polyvinylalkoholen zur Anwendung kommen.

Mit Vorteil ist der Aldehyd ein Polyaldehyd. Dieser ist vorzugsweise biologischen Ursprungs. Bei einer bevorzugten Ausführungsform der Zusammensetzung ist der Aldehyd ein oxidiertes Polysaccharid. Es ist besonders bevorzugt wenn sowohl das Aminogruppen tragende Polymer als auch der Aldehyd Polysaccharidgerüste aufweisen. Bei einer besonders bevorzugten Ausführungsform der Zusammensetzung ist der Aldehyd ein oxidiertes Polysaccharid, wobei das Polysaccharid mindestens eines aus der Gruppe von Dextran, Chitin, Stärke, Agar, Cellulose, Alginsäure, Glycosaminoglykane, Hyaluronsäure, Chondroitinsulfat und deren Derivate ist. Dextranaldehyd ist bevorzugt. Der Aldehyd, insbesondere der Dextranaldehyd, besitzt vorzugsweise ein Molekulargewicht von ca. 60.000 bis 600.000, insbesondere ca. 200.000. Höhere Molekulargewichte, insbesondere von mindestens 200.000, bringen hohe Vernetzungsgrade.

Vorteilhafterweise ist der Aldehyd teilweise oder vollständig maskiert. Zweck der Maskierung, insbesondere von oxidierten Polyaldehyden, ist es, die Bildung von intermolekularen Acetalen zu vermeiden und somit die Stabilität der Lösungen zu gewährleisten. Die kontrollierte Freisetzung der Aldehyde erfolgt schließlich in situ durch kontrollierte Hydrolyse in einem pH-Bereich von 2 bis 6, bevorzugt 2 bis 4,5. Es ist besonders bevorzugt, daß der Aldehyd mit einem S-, O- oder N-Nukleophil maskiert ist. Mit Vorteil ist der teilweise oder vollständig maskierte Aldehyd ein Polysaccharidalkalihydrogensulfitaddukt. Bei einer weiteren Ausführungsform der erfindungsgemäßen Zusammensetzung ist der Aldehyd teilweise oder vollständig mit Ethanol oder Glycerol maskiert.

Mit Vorteil sind die pH-Werte der Komponenten so abgestimmt, daß der pH-Wert einer Mischung der Komponenten zwischen 3 und 8, insbesondere zwischen 5 und 7,5 liegt. Ein hoher pH-Wert begünstigt zwar eine Vernetzung, führt aber zum Ausfallen von beispielsweise Chitosan.

Es ist insbesondere der Aldehyd, der für die Klebekraft verantwortlich ist und die Bindung an das Gewebe ermöglicht, jedoch ist allein durch den Aldehyd keine Bedeckung des Gewebes möglich. So ist in der erfindungsgemäßen Zusammensetzung die stöchiometrische Menge an Aldehydgruppen in Komponente b) mindestens die dreifache der stöchiometrischen Menge an Aminogruppen in Komponente a).

Vorteilhafterweise sind die Komponenten so aufeinander abgestimmt, daß sie nach Vereinigung in kurzer Zeit, insbesondere einer Zeit von 15 bis 200 Sekunden, miteinander vernetzen. Die Vemetzungszeit kann zum Beispiel durch die Konzentration der Lösungen und über das Mischungsverhältnis gesteuert werden. Der Vernetzungsgrad kann ebenfalls eingestellt werden, nämlich über die Zahl der Aldehydgruppen des Aldehyds.

Auch die Viskosität der Zusammensetzung ist steuerbar. Vorteilhafterweise sind die Viskositäten der Komponenten so aufeinander abgestimmt, daß eine Schicht der Zusammensetzung mit einer Dicke von 0,1 bis 1 mm applizierbar ist.

Die genannten Einstellungsmöglichkeiten (z.B. Vernetzungsgeschwindigkeit, Viskosität, Reaktivität) sind bei Zusammensetzungen mit Gelatine oder Kollagen, die je nach ihrem Ursprung verändert sind und keine definierten Reaktionen erlauben, nicht gegeben.

Vorteilhafterweise beträgt der Gehalt von Komponente b) an Aldehyd 5 bis 20 Gew.%, insbesondere 10 bis 15 Gew.%. Vorzugsweise beträgt der Gehalt von Komponente a) an aminogruppentragendem Polymer 1 bis 25 Gew. %, insbesondere 2 bis 20 Gew.%. Die Volumenverhältnisse der beiden Lösungen a) : b) liegen zwischen 5:1 und 1:5, vorzugsweise zwischen 3:1 und 1:3. Liegen sie, was in vielen Fällen bevorzugt ist, bei 1:1, dann können in einfacher Weise gleiche Volumina miteinander gemischt werden.

Bei einer besonders bevorzugten Ausführungsform der erfindungsgemäßen Zusammensetzung ist die Komponente a) eine essigsaure Lösung von Chitosan und Komponente b) eine wässrige Lösung von Dextranaldehyd. Dextran zeichnet sich beispielsweise gegenüber Glutaraldehyd (vgl. z.B. US 5,385,606) dadurch aus, dass es nicht toxisch ist.

Die Erfindung betrifft außerdem die Bereitstellung der erfindungsgemäßen Zusammensetzung zur Verwendung als chirurgischer Kleber, insbesondere zum Versiegelen oder Verschließen von Oberflächen bzw. Öffnungen. Bevorzugt werden die Komponenten kurz vor einer Applikation miteinander vermischt. Dies kann beispielsweise mit Hilfe einer Zwillingsspritze von statten gehen, bei der die beiden Komponenten in ein gemeinsames Auspressrohr, in dem sich ein statischer Mischer befindet, hineingedrückt werden. Durch den statischen Mischer im Auspressrohr werden die beiden Komponenten miteinander vermischt und werden, kurz bevor sie miteinander vernetzen, aus der Spritze auf die Applikationsstelle ausgepresst.

Es ist weiterhin auch möglich, daß die Komponenten erst auf einer Applikationsstelle vermischt werden, indem die beiden Komponenten beispielsweise kurz nacheinander auf eine Applikationsstelle aufgetragen werden.

Die Erfindung beansprucht auch einen Kit, bestehend aus zwei, bezogen auf den Inhalt, im wesentlichen getrennten Behältnissen, wobei jedes Behältnis jeweils eine Komponente der erfindungsgemäßen Zusammensetzung enthält. Bei einer bevorzugten Ausführungsform fungieren die beiden Behältnisse als Spritzenzylinder einer Doppelspritze. Bei einer solchen Doppelspritze, die auch Zwillingsspritze genannt wird, werden die getrennt gelagerten Komponenten in ein gemeinsames Auspressrohr gedrückt. Vorteilhafterweise weist der Kit eine Einrichtung zum Vermischen der Komponenten auf. Besonders bevorzugt ist es, daß der Kit einen statischen Mischer aufweist, der insbesondere auf eine Doppelspritze aufsteckbar ist. Dieser statische Mischer befindet sich insbesondere im Auspressrohr der Doppelspritze. Bei einer weiteren Ausführungsform ist die Doppelspritze an der Aufsteckstelle des Auspressrohres verschließ- und öffenbar.

Figurenbeschreibung

Figur 1 zeigt einen schematischen Längsschnitt durch eine bevorzugte Ausführungsform des erfindungsgemäßen Kits.

Die in der einzigen Zeichnung dargestellte bevorzugte Ausführungsform eines erfindungsgemäßen Kits zeigt einen Längsschnitt durch eine Doppelspritze 1. Diese Doppelspritze besteht aus zwei zusammenhängenden Spritzenzylindern 2a und 2b, welche die beiden Komponenten bei der erfindungsgemäßen Zusammensetzung getrennt beinhalten. Die Volumenverhältnisse der beiden Spritzenzylinder sind auf das Mischungsverhältnis der beiden Komponenten abgestimmt. Im vorliegenden Ausführungsbeispiel weisen die beiden Spritzenzylinder 2a und 2b gleiche Volumina an zwei Komponenten einer erfindungsgemäßen Zusammensetzung auf. Es ist auch möglich Doppelspritzen zu verwenden, bei denen die Volumina verschieden sind, beispielsweise die Zylinder verschieden große Durchmesser haben.

Ferner umfasst die Doppelspritze 1 zwei Spritzenstempel 3a und 3b, die durch eine Verbindungsplatte 4 miteinander verbunden sind. Am oberen Ende der beiden Spritzenstempel 3a und 3b sind jeweils zwei Kolbendichtringe 5a und 5b aufgebracht. Diese Kolbendichtringe schließen im wesentlichen luftdicht mit den Wandungen der beiden Spritzenzylinder 2a und 2b ab. An ihrem oberen Ende weist jeder der beiden Spritzenzylinder 2a und 2b jeweils eine, direkt aneinander angrenzende Öffnung 6a bzw. 6b auf. Diese Öffnungen sind bis zum ersten Gebrauch verschlossen.

Auf die beiden angrenzenden Öffnungen 6a und 6b ist nach deren Öffnen ein Auspressrohr 7 aufsteckbar. Im Auspressrohr 7 befindet sich ein statischer Mischer 8. An seinem oberen Ende verschmälert sich das Auspressrohr zu einer Auspressöffnung 9.

Beispielsweise durch Druck auf die Verbindungsplatte 4 und Gegendruck auf die Platte 10 bewegen sich die beiden Spritzenstempel 3a und 3b mit den daran befestigten Kolbendichtringen 5a und 5b in Richtung der Öffnungen 6a und 6b. Dadurch werden die beiden in den Spritzenzylindern befindlichen Komponenten aus den Öffnungen 6a und 6b in das Auspressrohr 7 gedrückt. Insbesondere durch den im Auspreßrohr 7 befindlichen statischen Mischer 8 werden die beiden Komponenten im Auspressrohr innig miteinander vermischt und werden schließlich in vermischtem Zustand aus der Auspressöffnung 9 auf eine Applikationsstelle gedrückt.

**<u>Beispiel einer erfindungsgemäßen Zusammensetzung:</u>**

1. Zusammensetzung

Lösung A: wässrige Lösung von Chitosan
Lösung B: wässrige Lösung von Dextranaldehyd

Durch Mischen der beiden Lösungen bildet sich ein Gel aus, welches adhäsive Eigenschaften besitzt. Die Gelierung beruht auf der Ausbildung von Iminen (Schiff'schen Basen) zwischen den Aldehydgruppen im oxidierten Dextran und den freien Aminogruppen im Chitosan (s. Reaktionsschema Anhang 1).

Alternativ zur Chitosanlösung können auch Lösungen von modifizierten Polysacchariden (mit Aminen modifiziertes Dextran) oder synthetischen Polymeren (mit Aminen modifizierter Polyvinylalkohol) verwendet werden.

1.1. Chitosanlösung

2g Chitosan werden in 100 ml 2%ige Essigsäurelösung (v/v) gegeben und fünf Tage bei Raumtemperatur gerührt.

Als Alternative hierzu wird eine 4 %ige wässrige (w/v) Protasan® UP Cl 213 (Fa. FMC Biopolymers, Drammen, Norwegen) Lösung (VE-Wasser) verwendet. Bei Protasan® UP Cl 213 handelt es sich um ein Chitosansalz mit Chlorid als Gegenion.

1.2 Synthese von Dextranaldehyd

Die zur Synthese verwendete 5%ige (w/v) Natriumperiodatlösung wird vor jeder Umsetzung frisch hergestellt und mit einer 10%igen (w/v) Dextranlösung vereinigt. Zur Herstellung der Dextranaldehyde können unterschiedliche stöchiometrische Verhältnisse verwendet (s. Tabelle 1) werden. Die Reaktionsmischung wird über Nacht bei Raumtemperatur gerührt, 2 Tage gegen destilliertes Wasser dialysiert und schließlich die aufgereinigte Reaktionslösung lyophilisiert. Das Reaktionsprodukt ist ein weißer faserartiger Feststoff.

Tabelle 1:

| Stöchiometrische Mengenverhältnisse der durchgeführten Synthesen | | | | |
|---|---|---|---|---|
| **Name** | **Molares Verhältnis $NaIO_4$: Dextraneinheit** | **Menge Dextranaldehydlösung** | **Menge $NaIO_4$-Lösung** | **Anteil an oxidierten Glucoseeinheiten (%)** |
| DA 3 | 3:5 | 300 ml<br>180 mmol | 460 ml<br>108 mmol | 30 |
| DA 4 | 4:5 | 300 ml<br>180 mmol | 612ml<br>144 mmol | 33 |
| DA 5 | 5:5 | 300 ml<br>180 mmol | 765 ml<br>180 mmol | 49 |
| DA 6 | 2:1 | 300 ml<br>180 mmol | 1430 ml<br>360 ml | 91 |
| DA 8 | 4:1 | 150 ml<br>90 mmol | 1430 ml<br>360 ml | 100 |

Von den auf die oben beschriebene Weise hergestellten Dextranaldehyden werden 15%ige Lösungen (w/v) hergestellt, indem 4,5g Dextranaldehyd in 30 ml destilliertes Wasser gegeben und über Nacht im Wasserbad bei 37°C geschüttelt werden. Für die Gelierung ist es vorteilhaft den pH-Wert der Dextranaldehydlösung durch Zugabe eines Phosphatpuffers zu erhöhen.

**Anteil an oxidierten Glucoseeinheiten im Dextran in Abhängigkeit vom molaren Verhältnis $NaIO_4$ pro Glucoseeinheiten**

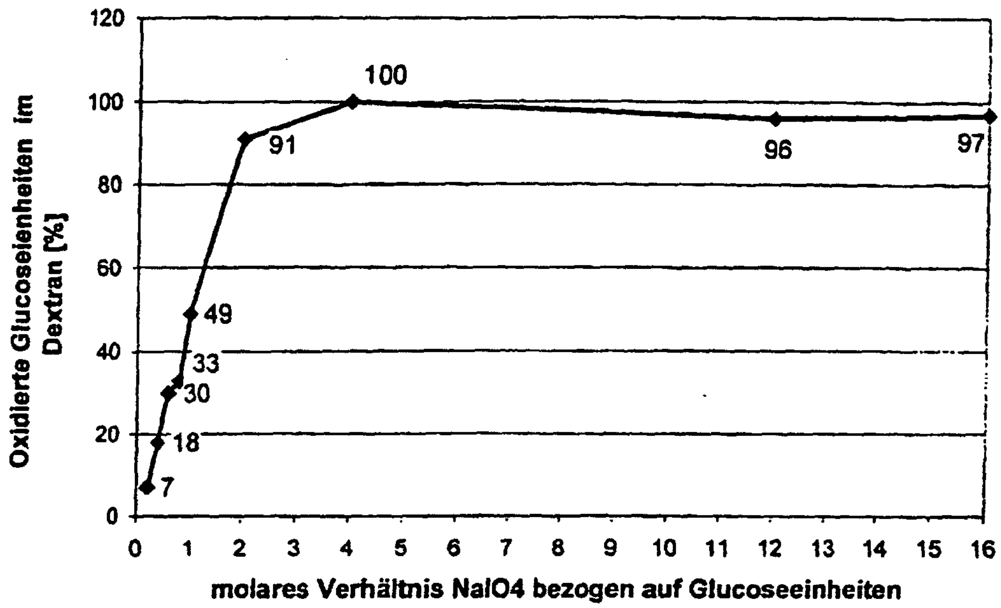


1.3 Molekulargewicht Dextran

Das mittlere Molekulargewicht im Dextran wurde variiert. Es wurde Dextran mit einem mittleren MW von 60.000 bis 90.000 Dalton (Fa. Fischer Scientific, Schwerte, Deutschland) und Dextran mit einem höheren mittleren MW von 413.263 Dalton (Fa. Sigma Aldrich Chemie GmbH, Steinheim, Deutschland) eingesetzt.

Das Molekulargewicht hatte keine Auswirkung auf den Anteil von oxidierten Glucoseeinheiten in Abhängigkeit von der eingesetzten Menge an $NaIO_4$.

Bestimmung des Aldehydgehalts

Die Bestimmung des prozentualen Anteils an oxidierten Glucoseeinheiten erfolgte titrimetrisch analog der Literatur [B.T. Hofreiter, B.H. Alexander, I.A. Wolff, Anal. Chem. 1955, 27, 1930ff.]

0,15 g Dextranaldehyd werden in einem Erlenmeyerkolben vorgelegt und anschließend mit 10 ml einer 0,25 N carbonatfreien NaOH Lösung versetzt. Die Mischung wird gerührt bis der eingesetzte Dextranaldehyd gelöst ist. Danach wird der Kolben für eine Minute in ein heißes Wasserbad (80°C) getaucht und anschließend unter starkem Rühren ins Eisbad gestellt. Nach einer Minute werden unter Rühren vorsichtig 15 ml 0,25 N Schwefelsäure hinzugegeben. Die Mischung wird anschließend mit 50 ml Wasser verdünnt und mit 1ml 0,2 %iger Phenolphthaleinlösung versetzt. Die saure Lösung wird mit 0,25 N NaOH Lösung gegen den Indikator titriert.

Aus der zugegebenen Menge an Dextran bzw. Dextranaldehyd sowie dem Verbrauch an Säure und Base berechnet sich der Dialdehydgehalt X wie folgt:

$$X = \left[ \frac{(n_{eqBase} - n_{eqSäure})_{DA}}{\frac{W_{DA}}{161}} - \frac{(n_{eqBase} - n_{eqSäure})_{Dextran}}{\frac{W_{Dextran}}{162}} \right] \times 100\%$$

X: Dialdehydgehalt
$n_{eqSäure}$: Äquivalentzstoffmenge der Säure
$n_{eqBase}$: Äquivalentstoffmenge der Base
$W_{DA}$: Trockengewicht Dextranaldehyd
$W_{Dextran}$: Trockengewicht Dextran
$n_{NaOH}$: Normalität des NaOH Titers
$n_{H2SO4}$: Normalität der verwendeten $H_2SO_4$-Lösung

In weiteren Syntheseansätzen wurde das optimale stöchiometrische Verhältnis $NaIO_4$ pro Glucoseeinheit Dextran ermittelt. Nachfolgende Grafik zeigt, dass ab einem stöchiometrischen Verhältnis $NaIO_4$ pro Glucoseeinheit Dextran von 2:1 der prozentuale Anteil an oxidierten Glucoseeinheiten über 90 % liegt.

2. Gelierungszeit der beiden Lösungen

Die Gelierungszeit hängt vom verwendeten Dextranaldehyd sowie vom Mischungsverhältnis der Dextranaldehyd- und der Chitosanlösung ab. Die Gelierungszeit nimmt mit zunehmendem Oxidationsgrad des Dextranaldehyds und mit zunehmendem Verhältnis 15%ige Dextranaldehydlösung: 2%ige Chitosanlösung zu. Sie liegt zwischen 15 und 200 Sekunden.

Tabelle 2:

| Gelierungszeiten in Abhängigkeit vom verwendeten Dextranaldehyd und vom Mischungsverhältnis der Lösungen | | |
|---|---|---|
| Dextranaldehyd | Verhältnis 2%ige Chitosan/15%ige Dextranal dehydlösung (ml) | |
| | 0,5/1,5 | 1,0/1,0 |
| DA 3 | 115 ± 31 s | 340 ± 56 s |
| DA 4 | 64 ± 10 s | 194 ± 54 s |
| DA 5 | 15 ± 2,9 s | 78 ± 33 s |
| DA 6 | 19 ± 2s | 15 ± 2s |

3. Bestimmung der Haftscherkraft

Die Haftscherkraft des neuen Gewebeklebers wird mit Hilfe von gereinigtem und lyophilisiertem Kollagen Typ I aus Rinderherzbeuteln (Lyoplant, BBraun Aesculap, Tuttlingen) bestimmt. Hierzu wird das Lyoplant zu Streifen mit 40 mm Länge und 10 mm Breite zugeschnitten, an deren Ende die zu verklebende Fläche von 1 $cm^2$ markiert wird. Die Klebung der Lyoplantstreifen verläuft wie folgt:
Die entsprechenden Mengenverhältnisse (s. Tabelle) Chitosan- und Dextranaldehydlösung werden in einem Reagenzglas vereinigt und 2 Sekunden geschüttelt, um eine gute Durchmischung der Lösungen zu erhalten. Anschließend werden jeweils 20 µl zentriert auf die zu verklebende Fläche aufgetragen. Die Klebefläche wird mit einer Folie vor dem Austrocknen geschützt und 10 Minuten mit 50g belastet. Danach werden die Streifen mit einer Zuggeschwindigkeit von 100 mm/min auseinandergezogen. Die Versuche wurden mit zwei unterschiedlichen Chargen Dextranaldehyd durchgeführt und der Mittelwert aus n = 13 Versuchen ermittelt. Die Ergebnisse der Versuche sind in Tabelle 3 bis 5 aufgelistet.

Tabelle 3:

| Vergleich der Haftscherkraft von DA 3 Charge 1 und 2 in Abhängigkeit vom Mischungsverhältnis 2%ige Chitosanlösung: 15%ige DA(3)Lösung | | |
|---|---|---|
| Volumenverhältnis 2%ige Chitosanlösung / 15%ige DA(3) Lösung | Haftscherkraft DA 3 Charge 1 (kPa) | Haftscherkraft DA 3 Charge 2 (kPa) |
| 3:1 | 121 ± 27,6 | 110 ± 27,6 |
| 1:1 | 167 ± 34,4 | 154 ± 25,7 |
| 1:3 | 137 ± 38,8 | 153 ± 33,5 |

Tabelle 4:

| Vergleich der Haftscherkraft von DA 4 Charge 1 und 2 in Abhängigkeit vom Mischungsverhältnis 2%ige Chitosanlösung zu 15%ige DA(4)Lösung | | |
|---|---|---|
| Volumenverhältnis 2%ige Chitosanlösung / 15%ige DA(4) Lösung | Haftscherkraft DA 4 Charge 1 (kPa) | Haftscherkraft DA 4 Charge 2 (kPa) |
| 3:1 | 128 ± 56 | 163 ± 56,8 |
| 1:1 | 124 ± 36,4 | 175 ± 22,2 |
| 1:3 | 167 ± 54,1 | 192 ± 71,8 |

Tabelle 5:

| Vergleich der Haftscherkraft von DA 5 Charge 1 und 2 in Abhängigkeit vom Mischungsverhältnis 2%ige Chitosanlösung zu 15%ige DA(5)Lösung | | |
|---|---|---|
| Volumenverhältnis 2%ige Chitosanlösung / 15%ige DA(5) Lösung | Haftkraft DA 5 Charge 1 (kPa) | Haftscherkraft DA 5 Charge 2 (kPa) |
| 3:1 | 136 ± 38,7 | 159 ± 37,1 |
| 1:1 | 148 ± 47,2 | 187 ± 42,9 |
| 1:3 | 223 ± 46 | 20,6 ± 41,2 |

Die Haftscherkraft nimmt ebenso wie die Gelierungszeit mit zunehmendem Oxidationsgrad und zunehmender Menge an Dextranaldehydlösung zu.

Analog zur Bestimmung der Haftscherkraft der Dextranaldehyd/Chitosanmischung wurden Untersuchungen mit Dextranaldehyd und einem Polyvinylalkohol-vinylaminpfropf-polymerisat ($PVALNH_2$) durchgeführt. Das Pfropfpolymerisat wurde als 20%ige, wässrige Lösung vom Hersteller geliefert und in unverdünntem Zustand zur Klebung von Lyoplantstreifen eingesetzt. Die Präparation der Lyoplantstreifen und die Auftragung der Lösungen wurden identisch zu den Dextranaldehyd/Chitosanklebungen durchgeführt.
Die Ergebnisse der Untersuchungen sind in den nachfolgenden Tabellen aufgelistet:

Tabelle 6:

| Vergleich der Haftscherkraft von DA 4 Charge 3 in Abhängigkeit vom Mischungsverhältnis 20%ige $PVALNH_2$-Lösung zu 15%ige DA(4) Lösung | |
|---|---|
| Volumenverhältnis (20 %) $PVALNH_2$-Lösung / 15%ige DA(4)Lösung | Haftscherkraft (kPa) |
| 3:1 | 155 ± 25,9 |
| 1:1 | 138 ± 29,0 |
| 1:3 | 159 ± 30,6 |

Tabelle 7:

| Vergleich der Haftscherkraft von DA 5 Charge 5 in Abhängigkeit vom Mischungsverhältnis 20%ige PVAL-$NH_2$-Lösung zu 15%ige DA(5)Lösung | |
|---|---|
| Volumenverhältnis PVAL$NH_2$-Lösung / 15%ige DA(5)Lösung | Haftscherkraft (kPa) |
| 3:1 | 145 ± 34,3 |
| 1:1 | 130 ± 19,0 |
| 1:3 | 198 ± 67,4 |

Zusätzliche Haftscherkraftuntersuchungen wurden mit höhermolekularem Dextranaldehyd und 4 % iger Protasanlösung durchgeführt. Die Lösungen wurden mit Hilfe eines Applikators der Firma Mixpac auf die Lyoplantstreifen aufgetragen. Der Applikator besteht aus einem Zweikammersystem mit aufgesetzter Mischerspitze. Das Lyoplant wurde zu Streifen mit einer Länge von 40 mm und einer Breite von 10 mm zugeschnitten, an deren Ende eine zu verklebende Fläche von 1 $cm^2$ markiert wurde. Die Lösungen wurden über den Mischer appliziert, die Klebefläche mit einer Folie abgedeckt, um diese vor dem Austrocknen zu schützen und 10 Minuten mit 50 g belastet. Danach wurden die Streifen mit einer Zuggeschwindigkeit von 100 mm/min. auseinandergezogen. Das mittlere MW des verwendeten Dextran beeinflusst die Haftscherkraft, wie in Tabelle 8 gezeigt wird:

Tabelle 8:

| Vergleich der Haftscherkräfte des neuen Klebers in Abhängigkeit vom mittleren Molekulargewicht des Dextranaldehyds. Mischungsverhältnis der Lösungen 1:1 | | |
|---|---|---|
| **Verwendetes DA** | **Choltosanlösung** | **Haftscherkraft (kPa)** |
| DA6 niedriges mittleres MW (15%ige Lösung) | 4%ige Protasanlösung | 188 +/- 38 |
| DA 6 hohes mittleres MW (15%ige Lösung) | 4%ige Protasanlösung | 278 +/- 71 |

Ebenso wurden Klebeversuche mit Bioglue® (Cryolife International Inc. USA) bestehend aus Proteinen und Glutaraldehyd sowie mit GLUETISS einem Gelatine-Resorcinol Dialdehydkleber, die gemäss der Gebrauchsanleitung auf die Lyoplantstreifen aufgetragen wurden, durchgeführt. Die mit diesen Klebern verklebten Streifen wurden ebenfalls mit einer Folie bedeckt und 10 Minuten mit 50 g belastet. Ein Vergleich der erzielten Haftscherkräfte ist in Tabelle 9 gezeigt.

Tabelle 9:

| Vergleich der Haftscherkraft einer erfindungsgemässen Zusammensetzung mit BioGlue® und GLUETISS® | | | |
|---|---|---|---|
| **Kleber** | **Zusammensetzung** | **Mischungsverhältnis** | **Haftscherkraft (kPa)** |
| Erfindungsgemässe Zusammensetzung | 4%ige Protasan Cl 213 und 15%ige DA 6 Lösung | 1/4 | 245 +/- 68 |
| Erfindungsgemässe Zusammensetzung | 4%ige Protasan Cl 213 und 15%ige DA 6 Lösung | 1/1 | 278 +/- 71 |
| Erfindungsgemässe Zusammensetzung | 4%ige Protasan Cl 213 und 15%ige DA 6 Lösung | 2/1 | 262 +/- 78 |
| Bioglue | Albuminlösung / Glutaraldehydlösung | 4/1 | 178 +/- 54 |
| Gluetiss | Gelatine Rescorcinol Lösung / Dialdehydlösung | gemäß Vorschrift | 167 +/- 37 |

4. Stillung von Leberblutungen

Der chirurgische Kleber wurde zur Stillung von Blutungen an der Rattenleber (SPF Wistar Ratten) eingesetzt.

Hierzu wurde eine Zusammensetzung aus einer 4 %igen wässrigen Protasanlösung und einer 15 %igen wässrigen Dextranaldehydlösung DA 6 gewählt. Die Lösungen wurden in einem Mischungsverhältnis von 1:1 eingesetzt. Zum Mischen und Auftragen der Komponenten wurde ein Applikator der Firma Mixpac verwendet.

Nach der Betäubung der Ratten wurde an der Leber das Modell der Kreuzinzision (Länge derr Schnitte: 2,5 cm) gewählt. Der Kleber wurde direkt nach der Inzision auf die blutende Wunde aufgetragen. Es bildete sich ein Gel aus, welches fest an der Leberoberfläche haftete, so dass die Blutung unmittelbar nach der Auftragung des Klebers zum Stillstand kam.

## Patentansprüche

1. Zusammensetzung aus mindestens zwei, insbesondere zwei, biokompatiblen, untereinander chemisch vernetzbaren Komponenten, insbesondere zum Verkleben von biologischem Gewebe, umfassend mindestens folgende Komponenten:

   a) wässrige Lösung mindestens eines aminogruppentragenden Polymers
   b) wässrige Lösung mindestens eines Aldehyds mit mindestens drei Aldehydgruppen,

   wobei die stöchiometrische Menge an Aldehydgruppen mindestens die dreifache der stöchiometrischen Menge an Aminogruppen im aminogruppentragenden Polymer ist und die Zusammensetzung frei von Eiweiß ist.

2. Zusammensetzung nach Anspruch 1, **dadurch gekennzeichnet, dass** der Aldehyd und das aminogruppentragende Polymer bei Körpertemperatur miteinander vernetzbar sind.

3. Zusammensetzung nach einem der Ansprüche 1 oder 2, **dadurch gekennzeichnet, dass** das aminogruppentragende Polymer von einem biologisch abbaubaren Naturstoff abgeleitet ist.

4. Zusammensetzung nach einem der vorhergehenden Ansprüche, **dadurch gekennzeichnet, dass** das aminogruppentragende Polymer ein Polysaccharid ist.

5. Zusammensetzung nach einem der vorhergehenden Ansprüche, **dadurch gekennzeichnet, dass** das aminogruppentragende Polymer Chitosan ist.

6. Zusammensetzung nach einem der vorhergehenden Ansprüche, **dadurch gekennzeichnet, dass** das aminogruppentragende Polymer ein mindestens teilweise deacetyliertes Chitin mit einem Deacetylierungsgrad von 50 bis 100 % ist.

7. Zusammensetzung nach einem der vorhergehenden Ansprüche, insbesondere nach Anspruch 1 oder 2, **dadurch gekennzeichnet, dass** das aminogruppentragende Polymer ein synthetisches Polymer ist.

8. Zusammensetzung nach Anspruch 7, **dadurch gekennzeichnet, dass** das synthetische Polymer ein modifizierter aminogruppentragender Polyvinylalkohol ist.

9. Zusammensetzung nach einem der vorhergehenden Ansprüche, **dadurch gekennzeichnet, dass** der Aldehyd ein Polyaldehyd ist.

10. Zusammensetzung nach einem der vorhergehenden Ansprüche, **dadurch gekennzeichnet, dass** der Aldehyd ein oxidiertes Polysaccharid ist.

11. Zusammensetzung nach Anspruch 10, **dadurch gekennzeichnet, dass** das Polysaccharid mindestens eines aus der Gruppe von Dextran, Chitin, Stärke, Agar, Cellulose, Alginsäure, Glykosaminoglykane, Hyaluronsäure, Chondroitinsulfat und deren Derivaten ist.

12. Zusammensetzung nach einem der vorhergehenden Ansprüche, **dadurch gekennzeichnet, dass** der Aldehyd, ein Molekulargewicht von 60.000 bis 600.000 besitzt.

13. Zusammensetzung nach einem der vorhergehenden Ansprüche, **dadurch gekennzeichnet, dass** der Aldehyd teilweise oder vollständig maskiert ist.

14. Zusammensetzung nach Anspruch 13, **dadurch gekennzeichnet, dass** der Aldehyd mit einem S-, O- oder N-Nucleophil maskiert ist.

15. Zusammensetzung nach einem der Ansprüche 13 oder 14, **dadurch gekennzeichnet, dass** der teilweise oder vollständig maskierte Aldehyd ein Polysaccharidalkalihydrogensulfitaddukt ist.

16. Zusammensetzung nach einem der Ansprüche 13 oder 14, **dadurch gekennzeichnet, dass** der Aldehyd teilweise oder vollständig mit Ethanol oder Glycerol maskiert ist.

17. Zusammensetzung nach einem der vorhergehenden Ansprüche, **dadurch gekennzeichnet, dass** der Gehalt an Komponente b) an Aldehyd 5 bis 20 Gew.-% beträgt.

18. Zusammensetzung nach einem der vorhergehenden Ansprüche, **dadurch gekennzeichnet, dass** der Gehalt von Komponente a) an aminogruppentragendem Polymer 1 bis 25 Gew.-% beträgt.

19. Zusammensetzung nach einem der vorhergehenden Ansprüche, **dadurch gekennzeichnet, dass** die pH-Werte der Komponenten so abgestimmt sind, dass der pH-Wert einer Mischung der Komponenten zwischen 3 und 8 liegt.

20. Zusammensetzung nach einem der vorhergehenden Ansprüche, **dadurch gekennzeichnet, dass** die Komponenten so aufeinander abgestimmt sind, dass sie nach Vereinigung in kurzer Zeit miteinander vernetzen.

21. Zusammensetzung nach einem der vorhergehenden Ansprüche, **dadurch gekennzeichnet, dass** die Viskositäten der Komponenten so aufeinander abgestimmt sind, dass eine Schicht der Zusammensetzung mit einer Dicke von 0,1 bis 1 mm applizierbar ist.

22. Zusammensetzung nach einem der Ansprüche 1 bis 6 und 9 bis 18, **dadurch gekennzeichnet, dass** Komponente a) eine essigsaure Lösung von Chitosan ist und Komponente b) eine wässrige Lösung von Dextranaldehyd ist.

23. Bereitstellung der Zusammensetzung nach einem der vorhergehenden Ansprüche zur Verwendung als chirurgischer Gewebekleber.

24. Bereitstellung nach Anspruch 23, **dadurch gekennzeichnet, dass** die Komponenten kurz vor einer Applikation miteinander vermischt werden können.

25. Bereitstellung nach Anspruch 23, **dadurch gekennzeichnet, dass** die Komponenten auf einer Applikationsstelle vermischt werden können.

26. Kit, bestehend aus zwei im wesentlichen getrennten Behältnissen, wobei die Behältnisse jeweils eine Komponente der Zusammensetzung nach einem der Ansprüche 1 bis 22 enthalten.

27. Kit nach Anspruch 26, **dadurch gekennzeichnet, dass** die beiden Behältnisse als Spritzenzylinder einer Doppelspritze fungieren.

28. Kit nach einem der Ansprüche 26 oder 27, **dadurch gekennzeichnet, dass** er eine Einrichtung zum Vermischen der Komponenten aufweist.

29. Kit nach einem der Ansprüche 26 bis 28, **dadurch gekennzeichnet, dass** er einen statischen Mischer aufweist.

## Claims

1. Composition of at least two, in particular two, biocompatible components which can be chemically crosslinked together, in particular for bonding biological tissue, comprising at least the following components:

   a) aqueous solution of at least one polymer having amino groups
   b) aqueous solution of at least one aldehyde having at least three aldehyde groups,

   where the stoichiometric amount of aldehyde groups is at least three times the stoichiometric amount of

amino groups in the polymer having amino groups and the composition is free of protein.

2. Composition according to Claim 1, **characterized in that** the aldehyde and the polymer having amino groups can be crosslinked together at body temperature.

3. Composition according to either of Claims 1 or 2, **characterized in that** the polymer having amino groups is derived from a biodegradable natural material.

4. Composition according to any of the preceding claims, **characterized in that** the polymer having amino groups is a polysaccharide.

5. Composition according to any of the preceding claims, **characterized in that** the polymer having amino groups is chitosan.

6. Composition according to any of the preceding claims, **characterized in that** the polymer having amino groups is an at least partially deacetylated chitin having a degree of deacetylation of from 50 to 100%.

7. Composition according to any of the preceding claims, in particular according to Claim 1 or 2, **characterized in that** the polymer having amino groups is a synthetic polymer.

8. Composition according to Claim 7, **characterized in that** the synthetic polymer is a modified polyvinyl alcohol having amino groups.

9. Composition according to any of the preceding claims, **characterized in that** the aldehyde is a polyaldehyde.

10. Composition according to any of the preceding claims, **characterized in that** the aldehyde is a oxidized polysaccharide.

11. Composition according to Claim 10, **characterized in that** the polysaccharide is at least one from the group of dextran, chitin, starch, agar, cellulose, alginic acid, glycosaminoglycans, hyaluronic acid, chondroitin sulfate and derivatives thereof.

12. Composition according to any of the preceding claims, **characterized in that** the aldehyde has a molecular weight of 60 000 to 600 000.

13. Composition according to any of the preceding claims, **characterized in that** the aldehyde is partially or completely masked.

14. Composition according to Claim 13, **characterized in that** the aldehyde is masked with an S, O or N nucleophile.

15. Composition according to either of Claims 13 or 14, **characterized in that** the partially or completely masked aldehyde is a polysaccharide-alkali metal hydrogensulfite adduct.

16. Composition according to either of Claims 13 or 14, **characterized in that** the aldehyde is partially or completely masked with ethanol or glycerol.

17. Composition according to any of the preceding claims, **characterized in that** the content of aldehyde of component b) is from 5 to 20% by weight.

18. Composition according to any of the preceding claims, **characterized in that** the content of polymer having amino groups of component a) is from 1 to 25% by weight.

19. Composition according to any of the preceding claims, **characterized in that** the pH values of the components are adjusted so that the pH of a mixture of the components is between 3 and 8.

20. Composition according to any of the preceding claims, **characterized in that** the components are adjusted with respect to one another so that they crosslink together in a short time after they are combined.

21. Composition according to any of the preceding claims, **characterized in that** the viscosities of the components are adjusted in relation to one another so that a layer of the composition with a thickness of from 0.1 to 1 mm can be applied.

22. Composition according to any of Claims 1 to 6 and 9 to 18, **characterized in that** component a) is a solution of chitosan in acetic acid, and component b) is an aqueous solution of dextranaldehyde.

23. Provision of Composition according to any of the preceding claims for use as surgical tissue adhesive.

24. Provision as set forth in Claim 23, **characterized in that** the components can be mixed together shortly before application.

25. Provision as set forth in Claim 23, **characterized in that** the components can be mixed on an application site.

26. Kit consisting of two substantially separate containers, where the containers each contain one component of the composition according to any of Claims 1 to 22.

27. Kit according to Claim 26, **characterized in that** the two containers function as syringe barrels of a double syringe.

28. Kit according to either of Claims 26 to 27, **characterized in that** it has a device for mixing the components.

29. Kit according to any of Claims 26 to 28, **characterized in that** it has a static mixer.

## Revendications

1. Composition faite d'au moins deux, en particulier de deux composants biocompatibles, réticulables du point de vue chimique l'un avec l'autre, en particulier en vue du collage de tissus biologiques, comprenant au moins les composants suivants:

   a) une solution aqueuse d'au moins un polymère portant des groupements amino,
   b) une solution aqueuse d'au moins un aldéhyde ayant au moins trois groupements aldéhyde,

   la quantité stoechiométrique de groupements aldéhyde étant au moins le triple de la quantité stoechiométrique de groupements amino dans le polymère portant des groupements amino et la composition étant exempte de protéine.

2. Composition selon la revendication 1, **caractérisée en ce que** l'aldéhyde et le polymère portant des groupements amino sont réticulables l'un avec l'autre à la température du corps.

3. Composition selon l'une quelconque des revendications 1 ou 2, **caractérisée en ce que** le polymère portant des groupements amino est dérivé d'une substance naturelle biodégradable.

4. Composition selon l'une quelconque des revendications précédentes, **caractérisée en ce que** le polymère portant des groupements amino est un polysaccharide.

5. Composition selon l'une quelconque des revendications précédentes, **caractérisée en ce que** le polymère portant des groupements amino est la chitosane.

6. Composition selon l'une quelconque des revendications précédentes, **caractérisée en ce que** le polymère portant des groupements amino est une chitine au moins partiellement déacétylée, ayant un degré de déacétylation de 50 à 100%.

7. Composition selon l'une quelconque des revendications précédentes, en particulier selon la revendication 1 ou 2, **caractérisée en ce que** le polymère portant des groupements amino est un polymère synthétique.

8. Composition selon la revendication 7, **caractérisée en ce que** le polymère synthétique est un alcool polyvinylique modifié portant des groupements amino.

9. Composition selon l'une quelconque des revendications précédentes, **caractérisée en ce que** l'aldéhyde est un polyaldéhyde.

10. Composition selon l'une quelconque des revendications précédentes, **caractérisée en ce que** l'aldéhyde est un polysaccharide oxydé.

11. Composition selon la revendication 10, **caractérisée en ce que** le polysaccharide est au moins un polysaccharide du groupe constitué du dextrane, de la chitine, d'amidon, de l'agar, de la cellulose, de l'acide alginique, de glycosaminoglycanes, de l'acide hyaluronique, du sulfate de chondroitine et de leurs dérivés.

12. Composition selon l'une quelconque des revendications précédentes, **caractérisée en ce que** l'aldéhyde possède un poids moléculaire de 60 000 à 600 000.

13. Composition selon l'une quelconque des revendications précédentes, **caractérisée en ce que** l'aldéhyde est partiellement ou complètement masqué.

14. Composition selon la revendication 13, **caractérisée en ce que** l'aldéhyde est masqué à l'aide d'un nucléophile S-, O- ou N-.

15. Composition selon l'une quelconque des revendications 13 ou 14, **caractérisée en ce que** l'aldéhyde partiellement ou complètement masqué est un produit d'addition d'hydrogénosulfite alcalin de polysaccharide.

16. Composition selon l'une quelconque des revendications 13 ou 14, **caractérisée en ce que** l'aldéhyde est partiellement ou complètement masqué à l'aide d'éthanol ou de glycérol.

17. Composition selon l'une quelconque des revendications précédentes, **caractérisée en ce que** la teneur du composant b) en aldéhyde est de 5 à 20% en poids.

18. Composition selon l'une quelconque des revendications précédentes, **caractérisée en ce que** la teneur du composant a) en polymère portant des groupements amino est de 1 à 25% en poids.

19. Composition selon l'une quelconque des revendications précédentes, **caractérisée en ce que** les valeurs de pH des composants sont adaptées au point que la valeur de pH d'un mélange des composants se situe entre 3 et 8.

20. Composition selon l'une quelconque des revendications précédentes, **caractérisée en ce que** les composants sont adaptés les uns aux autres de telle manière à ce qu'après combinaison ils se réticulent rapidement les uns aux autres.

21. Composition selon l'une quelconque des revendications précédentes, **caractérisée en ce que** les viscosités des composants sont adaptées les unes aux autres au point qu'une couche de la composition puisse être appliquée à raison d'une épaisseur de 0,1 à 1 mm.

22. Composition selon l'une quelconque des revendications 1 à 6 et 9 à 18, **caractérisée en ce que** le composant a) est une solution dans l'acide acétique de chitosane et **en ce que** le composant b) est une solution aqueuse d'aldéhyde de dextrane.

23. Mise à disposition de la composition selon l'une quelconque des revendications précédentes en vue de l'utilisation en tant qu'agents chirurgicaux de collage de tissus.

24. Mise à disposition selon la revendication 23, **caractérisée en ce que** les composants peuvent être mélangés juste avant application.

25. Mise à disposition selon la revendication 23, **caractérisée en ce que** les composants peuvent être mélangés à un emplacement d'application.

26. Kit, se composant de deux récipients pour l'essentiel séparés, les récipients contenant, à chaque fois, un composant de la composition selon l'une quelconque des revendications 1 à 22.

27. Kit selon la revendication 26, **caractérisé en ce que** les deux récipients fonctionnent en tant que cylindres d'injection d'une seringue double.

28. Kit selon l'une quelconque des revendications 26 ou 27, **caractérisé en ce qu'**il présente un dispositif en vue du mélange des composants.

29. Kit selon l'une quelconque des revendications 26 à 28, **caractérisé en ce qu'**il présente un mélangeur statique.

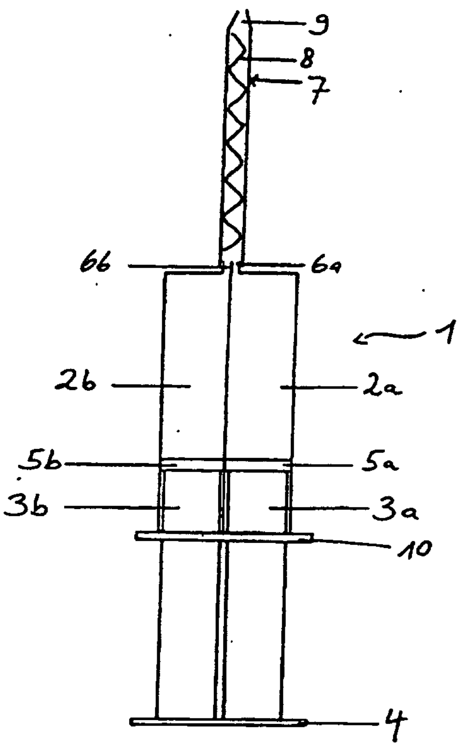


Figur 1